# EUROPEAN PATENT APPLICATION

(11) **EP 4 317 181 A1**
(43) Date of publication of application: **07.02.2024**
(21) Application number: 22859905.6
(22) Date of filing: 26.04.2022
(51) Int. Cl.: C07K 14/78, C12N 15/70, A23L 33/18, A61P 43/00, A61K 8/65, A61Q 19/00

(54) **RECOMBINANT TYPE-I HUMANIZED COLLAGEN POLYPEPTIDE, AND PREPARATION METHOD THEREFOR AND USE THEREOF**

(30) Priority: 23.08.2021 CN 202110968550
(71) Applicant: Shanxi Jinbo Bio-Pharmaceutical Co., Ltd., Shanxi 030032 (CN)
(72) Inventor: YANG, Xia, Taiyuan, Shanxi 030032 (CN); LU, Chenyang, Taiyuan, Shanxi 030032 (CN); LAN, Xiaobin, Taiyuan, Shanxi 030032 (CN); HE, Zhenrui, Taiyuan, Shanxi 030032 (CN); WANG, Jian, Taiyuan, Shanxi 030032 (CN); WANG, Lingling, Taiyuan, Shanxi 030032 (CN)
(74) Representative: Aera A/S
(86) International application number: PCT/CN2022/089283
(87) International publication number: WO 2023/024552

(57) **Abstract**

Disclosed in the present invention are a recombinant type-I humanized collagen polypeptide, and a preparation method therefor and the use thereof. The recombinant type-I humanized collagen polypeptide provided by the present disclosure comprises n repeats of a sequence as shown in SEQ ID NO: 1, wherein n is an integer greater than or equal to 1; the repetitive sequences are directly linked when n is an integer greater than or equal to 2; and optionally, the N-terminus of the recombinant type-I humanized collagen polypeptide contains an amino acid sequence capable of being cleaved off by the TEV protease. The recombinant type-I humanized collagen polypeptide provided by the present disclosure has an activity of promoting cell adhesion, the recombinant protein has an amino acid sequence selected from the amino acid sequence of natural collagen and does not generate immune response when applied to the human body, the preparation method therefor is simple, and high-yield collagen can be obtained at a low cost.

## Description

### PRIOROTY AND RELATED APPLICATION

The present application claims the priority of Chinese patent application No:202110968550.2 entitled "Recombinant Type-I Humanized Collagen Polypeptide, and Preparation Method therefor and Use thereof' filed on August 23, 2021, and the entire contents of this application, including appendix, are incorporated into herein by reference.

### TECHNICAL FIELD

The present disclosure pertains to the genetic engineering technical field, and particularly relates to a recombinant type-I humanized collagen polypeptide, a preparation method therefor and use thereof.

### BACKGROUND

Collagen is a kind of protein family, which is generally white, transparent and unbranched fibril. It is the basic support of skin and bones, accounting for 25% to 35% of the total protein. Collagen is mainly distributed in human skin, blood vessels, bones, tendons, teeth and cartilage, etc., and is the main matrix and scaffolding of these tissues, protecting and linking various tissues and playing an important physiological function in the body. Therefore, collagen can be widely used in industries such as medicine and cosmetics. According to the distribution and functional characteristics of collagen in the body, collagen can be divided into interstitial collagen, basement membrane collagen and pericellular collagen. Interstitial collagen molecules account for the vast majority of collagen in the entire body, including type-I, II and III collagen molecules. Type-I collagen is mainly distributed in tissues such as skin, tendons, and is most widely used in medicine.

However, natural collagen is insoluble in water and is inhomogeneous, so it is difficult to be directly used by the human body but often requires chemical treatment before it can be used. Moreover, the collagen products sold in the market at present are all taken from tissues of animals such as pigs, cattle and fish, so it is difficult to avoid the risk of viral infection, and meanwhile it may lead to immune rejection and allergic symptoms due to incompatibility with the human body. If collagen is extracted from a raw material of human placenta, not only is the source limited, but it also faces sever punishment by law. Therefore, at present, collagen can only be used in cosmetics and health products, and it cannot perform its original biological function at all.

The traditional method of producing collagen is to extract collagen derivatives by treating animal-derived tissues using acid, alkaline and enzymatic hydrolysis. The collagen extracted by these methods has itself lost its original biological activity and cannot be applied in the biomedical field to perform its real function. Most of the prepared collagen products have weak tensile strength, and pure collagen degrades quickly *in vivo*, which may have potential antigenicity, and due to the differences in sources, processing technologies and raw material ratios of the collagen, the nutritional components and feeding value of the products vary, and during processing, the leather is not only exposed to many chemicals but also susceptible to bacterial contamination, all of which seriously limit the application of collagen. Although foreign research institutions have obtained milk containing human collagen by breeding mice containing human collagen genes, the cost of such production is too high and the production cycle is too long to be put into mass production.

TEV Protease is a cysteine protease of Tobacco Etch Virus (TEV) with His tag (6×His tag) recombinantly expressed in *Escherichia coli,* and it can specifically recognize heptapeptide sequence Glu-Asn-Leu-Tyr-Phe-Gln-Gly/Ser and perform enzymatic cleavage between Gln and Gly/Ser amino acid residues, and is commonly used to remove Glutathione S-transferase(GST), His-tag or other tags of fusion proteins.

Therefore, it is imperative to find a product that can be rapidly absorbed by human body, is easy to prepare, is inexpensive, and at the same time can play the role in the functions and characteristics of human collagen.

### SUMMARY

### Technical problem

In response to the above-mentioned low collagen utilization caused by a series of problems in the art, such as low biological activity of heterologous collagen and its tendency to induce immune response, and high production cost, long cycle, and inability to mass produce human-derived collagen, the present disclosure provides a recombinant type-I humanized collagen polypeptide, and also provides a preparation method therefor and use thereof.

### Solution to problem

In the first aspect of the present disclosure, provided is a recombinant type-I humanized collagen polypeptide, which comprises n repeats of the sequence set forth in SEQ ID No: 1, n being an integer greater than or equal to 1, wherein the repetitive sequences are directly linked to each other when n is an integer greater than or equal to 2; optionally, the N-terminus of the recombinant type-I humanized collagen polypeptide comprises an amino acid sequence capable of being cleaved off by TEV protease.

Further, in the above-mentioned recombinant type-I humanized collagen polypeptide, the amino acid sequence capable of being cleaved off by TEV protease comprises a sequence set forth in SEQ ID No:4; preferably, the sequence set forth in SEQ ID No:4 is directly linked to the sequence set forth in SEQ ID No: 1.

Further, in the above-mentioned recombinant type-I humanized collagen polypeptide, the recombinant type-I humanized collagen polypeptide comprises: a. an amino acid sequence set forth in SEQ ID No:3; b. an amino acid sequence having 90%, 92%, 95%, 96%, 97%, 98% or 99% identity with the amino acid sequence set forth in SEQ ID No:3, which retains the cell adhesion effect of the amino acid sequence set forth in SEQ ID No:3; c. an amino acid sequence obtained by adding, substituting, deleting or inserting one or more amino acid residues into the amino acid sequence set forth in SEQ ID No:3, which retains the cell adhesion effect of the amino acid sequence set forth in SEQ ID No:3; or d. an amino acid sequence encoded by a nucleotide sequence which hybridizes with a polynucleotide sequence encoding the amino acid sequence set forth in SEQ ID No:3 under a stringency condition, and the amino acid sequence retains the cell adhesion effect of the amino acid sequence set forth in SEQ ID No:3, the stringency condition is a medium stringency condition, medium-high stringency condition, high stringency condition or very high stringency condition.

In the second aspect of the present disclosure, provided is a polynucleotide encoding the above-mentioned recombinant type-I humanized collagen polypeptide.

In the third aspect of the present disclosure, provided is an expression vector comprising the above-mentioned polynucleotide.

In the fourth aspect of the present disclosure, provided is a host cell comprising the above-mentioned expression vector.

In the fifth aspect of the present disclosure, provided is a method for producing the above-mentioned recombinant type-I humanized collagen polypeptide, which comprises the following steps: 1) culturing the host cell according to the fourth aspect of the present disclosure in a culture medium and producing a polypeptide; 2) harvesting and purifying the polypeptide, preferably purifying the polypeptide by Ni column and/or anion exchange chromatography; and 3) optionally enzymatically cleaving the polypeptide, preferably enzymatically cleaving the polypeptide with TEV protease.

In the sixth aspect of the present disclosure, provided is the use of the above-mentioned recombinant type-I humanized collagen polypeptide in the preparation of a product, wherein the product preferably is a tissue engineering product, a cosmetic, a health product or a medicine.

In the seventh aspect of the present disclosure, provided is a product comprising the above-mentioned recombinant type-I humanized collagen polypeptide, wherein the product preferably is a tissue engineering product, a cosmetic, a health product or a medicine.

In the eighth aspect of the present disclosure, provided is the use of the above-mentioned recombinant type-I humanized collagen polypeptide in the preparation of a product having a function of promoting cell adhesion.

### Advantageous effects of the disclosure

By the implementation of the above-mentioned technical solutions, the recombinant type-I humanized collagen prepared by the present disclosure has better cell adhesion effect as compared with other fragments of type-I collagen, and it will not produce immune response when applied to human body because its amino acid sequence is selected from amino acid sequence of natural collagen. Meanwhile, the preparation method is simple, and a high yield of collagen can be obtained at low cost.

### BRIEF DESCRIPTION OF THE DRAWINGS

FIG. 1 is a protein electrophoretogram after induced expression of the recombinant type-I humanized collagen TC1R4; the first lane is molecular weight Marker, and the second lane is purified TC1R4 protein; the molecular weight of TC1R4 protein detected by electrophoresis is about 38kDa, which corresponds to the protein comprising the amino acid sequence set forth in SEQ ID No:3.
FIG. 2 shows detection results of bio-adhesive activity of humanized collagen, C1S4T, C1S5T and TC1R4 protein polypeptides.

### DETAILED DESCRIPTION

Embodiments of the present disclosure are described below, but the present disclosure is not limited thereto. Unless otherwise specified, the instruments, reagents and materials used in the present disclosure are available by conventional commercial means.

In this specification, the use of "can" includes both the meaning of performing some processing and the meaning of not performing some processing. In this specification, "optional" or "optionally" means that the event or situation described next may or may not occur, and the description includes the situation where the event occurs and the situation where the event does not occur.

In this specification, "medical instrument" refers to instruments, equipment, appliances, in-vitro diagnostic reagents and calibrators, materials and other similar or related articles directly or indirectly used in human body.

In this specification, "tissue engineering product" refers to a product used in tissue engineering. Tissue engineering is an emerging discipline that combines cell biology and material science to construct tissues or organs *in vitro* or *in vivo.*

The present disclosure is based in part on the following finding of the inventors in the previous research: when expressing polypeptides comprising a plurality of repetitive sequences such as GEKGSPGADGPAGAPGTPGPQGIAGQRGVVGLPGQRGERGFPGLPGPSGEPGKQGPSG AS (SEQ ID No:1) in *Escherichia coli,* because the polypeptides are often truncated proteins, the hinge region structure of full-length proteins is lacking. Therefore, in order to improve the gelation of a recombinantly expressed polypeptide, it is often necessary to add sequences such as a hinge amino acid GPPGPCCGGG (SEQ ID No:2) to the C-terminus of the polypeptide to aid gelation (reference: Journal of Biochemistry.2004; 136:643-649). Therefore, in the invention application with the application number of CN201811438582.6, a polypeptide sequence comprising SEQ ID No:2 at the C-terminus, such as T16a, is designed, but it is found that in shake-flask or fermentation cultivation of polypeptides like the sequence of T16a, most of the target polypeptides form colloidal precipitates and cannot be dissolved and purified, resulting in low yields. In order to solve this problem, a polypeptide comprising multiple repetitive sequences is obtained by adding a non-collagen amino acid linker between the respective repetitive sequences (SEQ ID No:1), and the modification of this polypeptide is mainly focused on the linker amino acid residues and the amino acid in the C-terminal hinge region, also known as C-terminal stable sequence.

Unexpectedly, after resolving the crystal structure of SEQ ID No:1, the inventors found that the region of SEQ ID No:1 could form a very stable collagen trimer structure without the participation of a hinge region. In the invention application with the application number of CN201811438582.6, when the inventors continue to modify the polypeptide sequence, it is found that the polypeptide claimed by the above-mentioned application does not require the participation of the C-terminal stable sequence. Moreover, at this time, the polypeptide will not form a colloidal structure prematurely while being recombinantly expressed and affect the later protein purification.

In the present disclosure, the used repetitive sequence of SEQ ID No:1 is: GEKGSPGADGPAGAPGTPGPQGIAGQRGVVGLPGQRGERGFPGLPGPSGEPGKQGPSG AS (SEQ ID No:1). The polypeptide of the present disclosure can comprise multiple repetitive sequences set forth in SEQ ID No: 1, provided that there is no linker between the repetitive sequences. In this text, the linker can be one or more amino acid residues. In the present disclosure, there is no limitation on the number of the repetitive sequences as long as they can realize the characteristics verified in the adhesion examples. Preferably, the number of the repetitive sequences is 4, that is, a polypeptide comprising a sequence set forth in SEQ ID No:3: GEKGSPGADGPAGAPGTPGPQGIAGQRGVVGLPGQRGERGFPGLPGPSGEPGKQGPSG ASGEKGSPGADGPAGAPGTPGPQGIAGQRGVVGLPGQRGERGFPGLPGPSGEPGKQGP SGASGEKGSPGADGPAGAPGTPGPQGIAGQRGVVGLPGQRGERGFPGLPGPSGEPGKQ GPSGASGEKGSPGADGPAGAPGTPGPQGIAGQRGVVGLPGQRGERGFPGLPGPSGEPG KQGPSGAS (SEQ ID No:3).

When the polypeptide sequence of the present disclosure is expressed, an ENLYFQ (SEQ ID No:4) sequence should be added to its N-terminus, which can be cleaved off by TEV protease to directly obtain a polypeptide having the sequence set forth in SEQ ID No:3. preferably, the ENLYFQ (SEQ ID No:4) sequence is directly linked to the N-terminus of the first repetitive sequence, namely,

ENLYFQGEKGSPGADGPAGAPGTPGPQGIAGQRGVVGLPGQRGERGFPGLPG
PSGEPGKQGPSGASGEKGSPGADGPAGAPGTPGPQGIAGQRGVVGLPGQRGERGFPGL
PGPSGEPGKQGPSGASGEKGSPGADGPAGAPGTPGPQGIAGQRGVVGLPGQRGERGFP
GLPGPSGEPGKQGPSGASGEKGSPGADGPAGAPGTPGPQGIAGQRGVVGLPGQRGERG
FPGLPGPSGEPGKQGPSGAS (SEQ ID No:5).

In the research, the inventors found that the polypeptide having the sequence set forth in SEQ ID No:3 has higher activity than the recombinant type-I humanized collagen described in the invention application with the application number of CN201811254050.7.

In the present disclosure, the polypeptide is a recombinant type-I humanized collagen, which can be used herein interchangeably with the recombinant humanized collagen.

In the present disclosure, the recombinant humanized collagen can be prepared by conventional methods in the art. For example, it can be produced by the following steps: 1) construction of genetically engineered *Escherichia coli*; 2) fermentation cultivation of genetically engineered *Escherichia coli*; 3) induction and expression of recombinant humanized collagen; 4) purification and optional enzymatic cleavage of the recombinant humanized collagen.

In the above-mentioned step 1), the construction of genetically engineered *Escherichia coli* can be carried out by the following steps: a. obtaining a target gene fragment; b. inserting the obtained target gene fragment into a pET-32a expression vector to obtain a recombinant expression plasmid; c. transferring the recombinant expression plasmid into *Escherichia coli* competent cell BL21 (DE3), and screening to obtain positive genetically engineered *Escherichia coli.*

In the above-mentioned steps 2) and 3), fermentation cultivation of genetically engineered *Escherichia coli* and induction and expression of recombinant humanized collagen can be carried out by the following steps: a. selecting a single colony of the screened genetically engineered *Escherichia coli* from a transformed LB plate, and culturing the same in 1000 ml LB medium containing ampicillin at 37°C and 220 rpm until OD₆₀₀ of the bacterial solution reaches 0.9 to 1.1; b. adding IPTG having a final concentration of 0.5 mM for induction, culturing overnight at 18°C, and centrifuging to collect the bacteria.

In the above-mentioned step 4), the purification and enzymatic cleavage of the recombinant humanized collagen can be carried out by the following steps: a. resuspending the bacteria using a Tris buffer (25 mM Tris, 200 mM NaCl, pH 8.0), disrupting the bacteria, and centrifuging to collect the supernatant; b. combining the recombinant humanized collagen using a Ni₆FF affinity column, rinsing the impure proteins with a washing buffer containing 20 mM imidazole, eluting the target protein with a solution containing 250 mM imidazole, adding TEV protease to carry out enzymatic cleavage for 2 hours at 16°C, to finally obtain the target collagen polypeptide.

The host cell can be eukaryotic cells such as fungus and yeast, and prokaryotic cells such as Enterobacteriaceae bacteria. It should be understood that those skilled in the art can replace the above-mentioned *Escherichia coli* strain by other expression strains as a host cell.

The present disclosure further provides a nucleic acid molecule, which includes a nucleic acid sequence encoding the polypeptide of the present disclosure. The nucleic acid can be DNA or cDNA. The nucleic acid molecule can be mainly composed of the nucleic acid sequence encoding the peptide of the present disclosure, or may be composed only of the nucleic acid sequence encoding the peptide of the present disclosure. Such nucleic acid molecule can be synthesized by methods known in the art. Because of the degeneracy of the genetic code, those skilled in the art should understand that nucleic acid molecules having different nucleic acid sequences can encode the same amino acid sequence.

The present disclosure further provides a vector, which comprises the nucleic acid sequence of the present disclosure. Suitable vectors are known in the field of vector construction, including the selection of a promoter and other regulatory elements such as an enhancer element. The vector of the present disclosure comprises a sequence suitable for introduction into cells. For example, the vector can be an expression vector, in which the encoding sequence of the polypeptide is controlled by its own cis-acting regulatory element, and the vector is designed so as to facilitate gene integration or gene replacement of the host cell.

It should be understood by those skilled in the art that in the present disclosure, the term "vector" includes DNA molecules, such as plasmids, phages, viruses or other vectors, and it contains one or more heterologous or recombinant nucleic acid sequences. Suitable phage and virus vectors include, but are not limited to: λ-phage, EMBL phage, simian virus, bovine wart virus, Epstein-Barr virus, adenovirus, herpes virus, mouse sarcoma virus, mouse breast cancer virus, lentivirus, etc.

The polypeptide of the present disclosure comprises the sequence set forth in SEQ ID No:3 or a sequence obtained by substituting, deleting, inserting and/or adding one or more amino acids in the sequence set forth in SEQ ID No:3, as long as the polypeptide of the present disclosure retains the cell adhesion effect of the amino acid sequence set forth in SEQ ID No:3. "More" can be 2, 3, 4, 5, 6, 7, 8, 9, 10 or 11.

Amino acid addition refers to the addition of amino acids to the C-terminus or N-terminus of an amino acid sequence, such as SEQ ID No:3, as long as the polypeptide of the present disclosure retains the cell adhesion effect of the amino acid sequence set forth in SEQ ID No:3.

Amino acid substitution refers to that some amino acid residue at a certain position in an amino acid sequence, such as the sequence set forth in SEQ ID No:3, is substituted by another amino acid residue, as long as the polypeptide of the present disclosure retains the cell adhesion effect of the amino acid sequence set forth in SEQ ID No:3.

Amino acid insertion refers to the insertion of amino acid residues to appropriate positions of an amino acid sequence, such as the sequence set forth in SEQ ID No:3, and the inserted amino acid residues can be all or partially adjacent to each other, or none of the inserted amino acids are adjacent to each other, as long as the polypeptide of the present disclosure retains the cell adhesion effect of the amino acid sequence set forth in SEQ ID No:3. In this text, the insertion positions of the amino acids are not between the repetitive sequences.

Amino acid deletion refers to that 1, 2, 3 or more amino acids can be deleted from an amino acid sequence, such as the sequence set forth in SEQ ID No:3, as long as the polypeptide of the present disclosure retains the cell adhesion effect of the amino acid sequence set forth in SEQ ID No:3.

In the present disclosure, substitution can be conservative amino acid substitution, which means that as compared with the amino acid sequence set forth in SEQ ID No:3, three, more preferably two or one amino acid is substituted with an amino acid having similar properties to form a peptide. These conservative mutant peptides can be produced by amino acid substitution according to the following table.

| Original residue | Representative substitution | Preferable substitution |
|---|---|---|
| Ala(A) | Val;Leu;Ile | Val |
| Arg(R) | Lys;Gln;Asn | Lys |
| Asn(N) | Gln;His;Lys;Arg | Gin |
| Asp(D) | Glu | Glu |
| Cys(C) | Ser | Ser |
| Gln(Q) | Asn | Asn |
| Glu(E) | Asp | Asp |
| Gly(G) | Pro;Ala | Ala |
| His(H) | Asn;Gln;Lys;Arg | Arg |
| Ile(I) | Leu;Val;Met;Ala;Phe | Leu |
| Leu(L) | He; Vai; Met; Ala; Phe | Ile |
| Lys(K) | Arg;Gln;Asn | Arg |
| Met(M) | Leu;Phe;Ile | Leu |
| Phe(F) | Leu; Val; Ile ; Ala; Tyr | Leu |
| Pro(P) | Ala | Ala |
| Ser(S) | Thr | Thr |
| Thr(T) | Ser | Ser |
| Trp(W) | Tyr; Phe | Tyr |
| Tyr(Y) | Trp; Phe; Thr; Ser | Phe |
| Val(V) | Ile;Leu;Met;Phe;Ala | Leu |

As used herein, the terms "medium stringency condition", "medium-high stringency condition", "high stringency condition" or "very high stringency condition" describe conditions for hybridization and washing of nucleic acids. For guidance on performing hybridization reactions, see Current Protocols in Molecular Biology, John Wiley & Sons, N.Y. (1989), 6.3.1 to 6.3.6, which is incorporated herein by reference. Both aqueous and non-aqueous methods are described in this literature, and either can be used. For example, the specific hybridization conditions are as follows: 1) the low stringency hybridization conditions are washing twice in 6 × sodium chloride/sodium citrate (SSC) at about 45°C and then at 50°C or higher in 0.2×SSC and 0.1 %SDS (the washing temperature can be raised to 55°C for low stringency hybridization conditions); 2) the medium stringency hybridization conditions are washing once or more in 6 × SSC at about 45°C and then at 60°C in 0.2×SSC and 0.1 % SDS; 3) the high stringency hybridization conditions are washing once or preferably washing more than once in 6 × SSC at about 45°C and then at 65°C in 0.2×SSC and 0.1 % SDS; 4) the very high stringency hybridization conditions are washing once or more in 0.5M sodium phosphate, 7% SDS, at 65°C and then at 65°C in 0.2×SSC and 1% SDS.

In practical applications, the polypeptide of the present disclosure or pharmaceutical salts thereof, derivatives thereof or pharmaceutical salts of the same, the above-mentioned conjugate, the above-mentioned polymer and the above-mentioned composition can be directly administered to patients as drugs, or mixed with appropriate carriers or excipients and administered to patients. Materials of the carriers here include, but are not limited to, water-soluble carrier materials (e.g., polyethylene glycol, polyvinylpyrrolidone, organic acids, etc.), insoluble carrier materials (e.g., ethyl cellulose, cholesterol stearate, etc.), and enteric carrier materials (e.g., cellulose acetate phthalate and carboxymethyl cellulose, etc.). Among them, water-soluble carrier materials are preferrable. A variety of dosage forms can be made from these materials, including but not limited to tablet, capsule, dripping pill, aerosol, pill, powder, solution, suspension, emulsion, granule, liposome, transdermal agent, buccal tablet, suppository, lyophilized powder for injection, etc. Among them, the suppository can be vaginal suppository, vaginal ring, or ointment, cream or gel suitable for vaginal application. The polypeptide dosage form can be common preparations, sustained-release preparations, controlled-release preparations and various microparticle drug delivery systems. In order to produce the unit dosage form into tablets, various carriers known in the art can be widely used. Examples of the carriers are, for example, a diluent and an absorbent such as starch, dextrin, calcium sulfate, lactose, mannitol, sucrose, sodium chloride, glucose, urea, calcium carbonate, kaolin, microcrystalline cellulose, aluminum silicate, etc.; a wetting agent and an adhesive, such as water, glycerol, polyethylene glycol, ethanol, propanol, starch paste, dextrin, syrup, honey, glucose solution, arabic gum paste, gelatin paste, sodium carboxymethyl cellulose, lac, methyl cellulose, potassium phosphate, polyvinylpyrrolidone, etc.; a disintegrator, such as dried starch, alginate, agar powder, brown algae starch, sodium bicarbonate and citric acid, calcium carbonate, polyoxyethylene, sorbitol fatty acid ester, sodium dodecyl sulfonate, methyl cellulose, ethyl cellulose, etc.; a disintegration inhibitor, such as sucrose, glyceryl tristearate, cocoa butter, hydrogenated oil, etc.; an absorption promoter, such as quaternary ammonium salt, sodium dodecyl sulfate, etc.; a lubricant, such as talcum powder, silica, corn starch, stearate, boric acid, liquid paraffin, polyethylene glycol, etc. The tablets can also be further produced into coated tablets, such as sugar coated tablets, film coated tablets, enteric coated tablets, or double-layer tablets and multi-layer tablets. In order to produce the unit dosage form into pills, various carriers known in the art can be widely used. Examples of the carriers are, for example, a diluent and an absorbent such as glucose, lactose, starch, cocoa butter, hydrogenated vegetable oil, polyvinylpyrrolidone, Gelucire, kaolin, talcum powder, etc.; a binder such as Arabic gum, tragacanth gum, gelatin, ethanol, honey, liquid sugar, rice paste or batter, etc.; a disintegrator, such as agar powder, dried starch, alginate, sodium dodecyl sulfonate, methyl cellulose, ethyl cellulose, etc. In order to produce the unit dosage form into suppository, various carriers known in the art can be widely used. Examples of the carriers are, for example, polyethylene glycol, lecithin, cocoa butter, higher alcohols, esters of higher alcohols, gelatin, semi-synthetic glycerides, etc. In order to produce the unit dosage form into preparations for injection, such as solution, emulsion, lyophilized powder injection and suspension, all diluents commonly used in the art can be used, such as water, ethanol, polyethylene glycol, 1,3-propanediol, ethoxylated isostearyl alcohol, polyoxidized isostearyl alcohol, polyoxyethylene sorbitan fatty acid ester, etc. Besides, in order to prepare an isotonic injection, an appropriate amount of sodium chloride, glucose or glycerol can be added to the preparation for injection, and additionally, a conventional cosolvent, buffer, pH regulator and the like can be also added. In addition, if necessary, colorants, preservatives, spices, corrective agents, sweeteners or other materials can also be added to the pharmaceutical preparations.

The above-mentioned dosage forms can be administered by injection, including subcutaneous injection, intravenous injection, intramuscular injection and intraperitoneal injection, intracisternal injection or infusion, etc.; intracavitary administration, such as rectal, vaginal and sublingual administration; respiratory administration, such as intranasal administration; mucosal administration. Among the above-mentioned administration routes, injection is preferable, and a preferable injection route is subcutaneous injection.

The dosage of the polypeptide of the present disclosure or pharmaceutical salts thereof, derivatives thereof or pharmaceutical salts of the same, the above-mentioned conjugate, the above-mentioned polymer and the above-mentioned composition depends on many factors, such as the nature and severity of the disease to be prevented or treated, the gender, age, weight and individual reaction of the patient or animal, the specific active ingredients used, the route and times of administration, etc. The above-mentioned dosage can be administered in a single dosage form or divided into several dosage forms, such as two, three or four dosage forms. For any specific patient, the specific therapeutically effective dose level shall be based on a variety of factors. The factors include the disorder treated and the severity of the disorder; the activity of the specific active ingredients adopted; the specific composition adopted; the age, weight, general health status, gender and diet of the patient; administration time, administration route and excretion rate of the specific active ingredients adopted; duration of treatment; drugs used in combination with or at the same time with the specific active ingredients adopted; and similar factors known in the medical field. For instance, it is the practice in the art to start with a dose of the active ingredient at a level lower than that required to obtain the desired therapeutic effect and gradually increase the dose until the desired effect is obtained.

The human collagen C1S4T and the human collagen C1S5T mentioned in the present disclosure are described in the patent application No. CN201811254050.7 and entitled "polypeptide, production method therefor and application thereof.

In order to express the technical solution of the present disclosure more clearly, the following is a further description in combination with specific examples but cannot be used to limit the present disclosure, and this is only a part of examples of the present disclosure.

### Example 1: Preparation of recombinant type-I humanized collagen TC1R4

### 1. Construction of TC1R4 gene expression vector

The full-length protein sequence of recombinant type-I humanized collagen TC1R4 used in this example was the amino acid sequence set forth in SEQ ID No:3, with a full length of 240aa. In order to purify the TC1R4 protein after it was expressed, the amino acid sequence set forth in SEQ ID No:4 was linked to the N-terminus of the amino acid sequence set forth in SEQ ID No:3 to form an amino acid sequence set forth in SEQ ID No:5, which had a full length of 246aa. The encoding sequence corresponding to the amino acid sequence was optimized according to the codon of *Escherichia coli,* and the full length of the corresponding gene was 738bp (see the underlined sequence in SEQ ID No:6 for details). In order to facilitate the subsequent construction of expression vectors, a restriction site sequence was added at the 5' terminus of the encoding sequence, and a stop codon sequence and a restriction site sequence were added at the 3' terminus. The optimized gene sequence was as follows:

GGTACCGAAAACCTGTATTTCCAGGGTGAAAAAGGTAGTCCGGGTGCAGATGGTCC
GGCCGGTGCACCTGGTACACCGGGTCCTCAGGGCATTGCAGGCCAGCGTGGTGTTG
TGGGCCTGCCGGGTCAGCGCGGTGAACGTGGTTTTCCGGGTCTGCCGGGTCCGAGC
GGCGAACCTGGTAAACAGGGCCCGAGTGGTGCAAGCGGTGAAAAAGGCAGTCCG
GGCGCAGATGGTCCTGCAGGTGCCCCTGGTACACCTGGTCCGCAGGGTATTGCAGG
TCAGCGCGGCGTGGTGGGCCTGCCTGGTCAACGTGGCGAACGTGGTTTCCCGGGC
CTGCCGGGACCGTCAGGTGAACCTGGCAAACAGGGCCCTAGCGGTGCAAGTGGCG
AAAAAGGTAGCCCGGGCGCAGACGGCCCGGCAGGTGCACCTGGCACACCTGGTCC
TCAGGGTATTGCGGGCCAGCGCGGCGTTGTTGGTCTGCCTGGCCAGCGTGGCGAAC
GCGGTTTTCCGGGCCTGCCTGGCCCGTCAGGCGAACCTGGAAAACAGGGCCCAAG

TGGTGCAAGTGGTGAAAAAGGAAGTCCGGGCGCCGATGGCCCGGCCGGTGCGCCT
GGTACGCCTGGTCCTCAAGGCATTGCCGGTCAGCGCGGAGTTGTTGGCCTGCCGGG
CCAGCGTGGAGAACGCGGTTTCCCGGGTCTGCCTGGTCCGAGTGGTGAACCGGGT
AAACAGGGTCCGAGCGGTGCATCATAACTCGAG (SEQ ID No:6).

Beijing Shengyuan Kemeng Gene Biotechnology Co., Ltd. was entrusted to synthesize the gene fragment, and the synthesized TC1R4 gene fragment was inserted into a pET-32a expression vector (EMD Biosciences (Novagen) Company) via restriction sites of *KpnI* (NEB Company, item No:R0136L) and *XhoI* (NEB Company, item No:R0146L) to obtain a corresponding recombinant expression plasmid.

### 2. Transformation of recombinant expression plasmid

The expression plasmid successfully constructed above was transformed into *Escherichia coli* competent cell BL21(DE3) (Merck Company). The specific process was as follows: 1) 1 µL of the plasmid was put into 100 µL of *Escherichia coli* competent cell BL21(DE3), which was placed on ice for 30 minutes; 2) the mixture was subject to heat shock in a water bath at 42°C for 90 seconds and then was quickly placed on ice for 2 minutes; 3) 700 µL of non-resistant LB medium(10 g/L peptone, 5 g/L yeast extract and 10 g/L sodium chloride) was added to the mixture, followed by culture at 37°C and 220 rpm for 1 hour; 4) 200 µL of the bacterial solution was evenly coated on a LB agar plate (10 g/L peptone, 5 g/L yeast extract, 10 g/L sodium chloride, 15 g/L agar, 100 µg/mL ampicillin) containing ampicillin; 5) the plate was cultured upside down in an incubator at 37°C for about 16 hours until the grown colonies were clearly visible.

### 3. Induced expression of recombinant type-I humanized collagen TC1R4

Monoclonal colonies were selected from the transformed LB agar plate and cultured in 1000 ml LB (containing 100 µg/mL ampicillin) medium at 37°C and 220rpm until the OD₆₀₀ of the bacterial solution reached 0.9 to 1.1, and IPTG (Sigma Company, article No:I5502-1G) was added to have a final concentration of 0.5 mM for induction of the expression, and the induction condition was overnight culture at 18°C and 220rpm. Finally, the bacteria were collected by centrifugation and stored at -20°C or immediately subject to the next step of purification.

### 4. Purification of the recombinant type-I humanized collagen TC1R4

The above-mentioned bacterial precipitate was re-suspended(in 1L flask) with about 100 ml Tris buffer (25 mM Tris, 200 mM sodium chloride, pH 8.0), and the bacteria was broken with a homogenizer (GEA), and centrifuged at 17000 rpm for 30 minutes, to fully separate the soluble protein from the inclusion body.

An affinity column of Ni₆FF (Cytiva Company, article No:30210) was equilibrated with 5 times the column volume of a binding buffer (25 mM Tris, 200 mM NaCl, pH 8.0). Then the protein supernatant was added to fully bind the target recombinant protein to the column material. Subsequently, the impurity protein was rinsed with a washing buffer (20 mM imidazole, 25 mM Tris, 200 mM NaCl, pH 8.0) containing 20 mM imidazole (Sigma Company), and the target protein was eluted with a solution (250 mM imidazole, 25 mM Tris, 200 mM NaCl, pH 8.0) containing 250 mM imidazole. Then, appropriate amount of TEV protease (Sigma, T4455) was added, and incubation was carried out at 16°C for 2 hours. The filtrate was collected, which was the target collagen with the carrier protein removed. The obtained product was dialyzed overnight and lyophilized into dry powder for later use.

### 5. Purity detection of the recombinant type-I humanized collagen TC 1R4

The purity of the obtained TC1R4 protein was detected using SDS-PAGE. The specific process was as follows: 20 µL of purified protein solution was taken, to which 5 µl of 5× protein loading buffer (250 mM Tris-HCl (pH 6.8), 10% SDS, 0.5% bromophenol blue, 50% glycerol, 5% β-mercaptoethanol) was added, and the mixture was placed in boiling water at 100°C for 5 minutes, and then 10 µl was added to SDS-PAGE protein gel each well; after electrophoresis at 150V for 1 hour, the protein was dyed with Coomassie brilliant blue staining solution (0.1% Coomassie brilliant blue R-250, 25% ethanol, 10% glacial acetic acid) for 3 minutes, and then decolorized with a protein decolorizing solution (10% acetic acid, 5% ethanol).

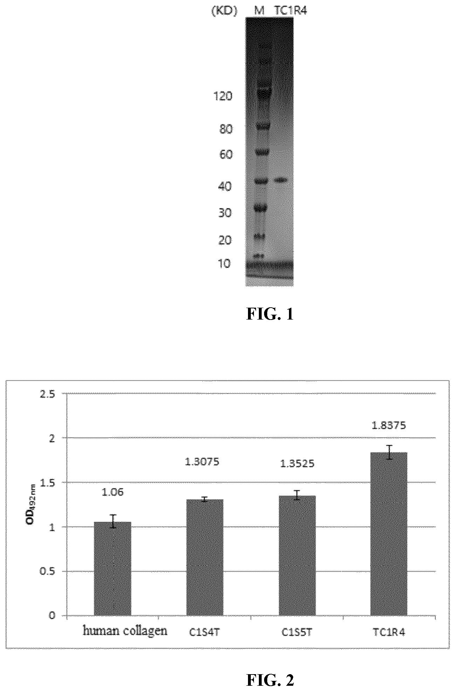

The results were shown in FIG. 1. The apparent molecular weight of TC1R4 obtained by electrophoresis was 38 kDa, which was corresponding to the polypeptide of TC1R4, indicating that the polypeptide TC1R4 was correctly expressed.

### Example 2: Mass spectrometry detection of recombinant type-I humanized collagenTC1R4

The mass spectrometry detection of TC1R4 protein was carried out according to the conditions shown in Table 1.

**Table 1 Conditions for mass spectrometry detection**

| **Instrument name** | Matrix-assisted laser desorption ionization time-of-flight mass spectrometry MALDI-TOF/TOF Ultraflextreme^{™}, Brucker, Germany | | |
|---|---|---|---|
| **Matrix** | CHCA | **Laser energy** | 125 |
| **Data retrieval** | Mascot | **Retrieved** | ALL entries or human |
| **software** | | **species** | |
| **Retrieval database** | Swissprot | | |

After undergoing DTT reduction and iodoacetamide alkylation, a TC1R4 protein sample was enzymatically hydrolyzed overnight by adding trypsin. The peptide fragments obtained after the enzymolysis were desalted by C18ZipTip, and then mixed with the matrix α-Cyano-4-hydroxycinnamic acid (CHCA) on a plate. Finally, analysis was carried out using matrix-assisted laser desorption ionization time-of-flight mass spectrometry MALDI-TOF/TOF UltraflextremeTM, Brucker, Germany (see Protein J.201635:212-7 for the technology of peptide mass fingerprint).

Database retrieval was handled from the webpage of Peptide Mass Fingerprint on the local mascot website. Protein identification results were obtained according to the primary mass spectrometry of the peptide fragments produced after enzymolysis. Retrieval parameters: Trypsin enzymolysis, 2 missed cleavage sites. The alkylation of cysteine was set as a fixed modification and the oxidation of methionine as a variable modification. The database used for the identification was Swissprot.

The molecular weight and corresponding polypeptides detected by mass spectrometry are shown in Table 2.

**Table 2 Molecular weight and corresponding polypeptides detected by mass spectrometry**

| Observed value | Mr (predictive value) | Mr (calculated value) | Peptides |
|---|---|---|---|
| 2095.0960 | 2094.0888 | 2093.9339 | ADGAILVDFWAEWCGPCK (SEQ ID NO:7) |
| 2537.3652 | 2536.3579 | 2536.2306 | GERGFPGLPGPSGEPGKQGPSGASGEK (SEQ ID NO:8) |
| 2195.1865 | 2194.1792 | 2194.0655 | GFPGLPGPSGEPGKQGPSGASGEK (SEQ ID NO:9) |
| 4249.1329 | 4248.1256 | 4248.0585 | GERGFPGLPGPSGEPGKQGPSGASGEKGSPGAD GPAGAPGTPGPQGIAGQR (SEQ ID NO:10) QGPSGASGEKGSPGADGPAGAPGTPGPQGIAGQ R(SEQ ID NO:11) |
| 2971.5627 | 2970.5554 | 2970.4180 | |
| 2073.0969 | 2072.0896 | 2072.0036 | GSPGADGPAGAPGTPGPQGIAGQR (SEQ ID NO:12) |
| 882.5634 | 881.5561 | 881.5083 | GWGLPGQR (SEQ ID NO:13) |
| 1224.7446 | 1223.7373 | 1223.6735 | GWGLPGQRGER (SEQ ID NO:14) |
| 1638.9088 | 1637.9015 | 1637.8162 | GERGFPGLPGPSGEPGK (SEQ ID NO:15) |
| 2223.1981 | 2222.1909 | 2222.0716 | GERGFPGLPGPSGEPGKQGPSGAS (SEQ ID NO: 16) |
| 1296.7342 | 1295.7269 | 1295.6510 | GFPGLPGPSGEPGK (SEQ ID NO:17) |
| 1880.9932 | 1879.9859 | 1879.9065 | GFPGLPGPSGEPGKQGPSGAS (SEQ ID NO:18) |

As compared with the sequence of polypeptide:

GEKGSPGADGPAGAPGTPGPQGIAGQRGVVGLPGQRGERGFPGLPGPSGEPG
KQGPSGASGEKGSPGADGPAGAPGTPGPQGIAGQRGVVGLPGQRGERGFPGLPGPSGE
PGKQGPSGASGEKGSPGADGPAGAPGTPGPQGIAGQRGVVGLPGQRGERGFPGLPGPS
GEPGKQGPSGASGEKGSPGADGPAGAPGTPGPQGIAGQRGVVGLPGQRGERGFPGLPG
PSGEPGKQGPSGAS (SEQ ID NO:3)

the coverage rate of the peptide fragments was 98.75% as calculated, and the detection result was highly reliable.

### Example 3: Biological activity detection of recombinant type-I humanized collagen TC1R4

For the detection method of collagen activity, please refer to document Juming Yao, Satoshi Yanagisawa, Tetsuo Asakura, Design, Expression and Characterization of Collagen-Like Proteins Based on the Cell Adhesive and Crosslinking Sequences Derived from Native Collagens, J Biochem. 136, 643-649(2004). The specific implementation method was as follows.
1) An UV absorption method was used to detect the concentration of the protein samples, including commercialized human collagen (Sigma, C7774), the recombinant type-I humanized collagen TC1R4 provided by the present disclosure, recombinant type-III collagen C1S4T and recombinant type-III collagen C1S5T (wherein the sequences of the proteins C1S4T and C1S5T are referred to in patent application No. CN201811254050.7).
   Specifically, the UV absorption of the samples at 215 nm and 225 nm were measured separately, and the protein concentration was calculated using the empirical formula C(µg/mL)= 144×(A215-A225), noting that the detection should be performed when A215<1.5. The principle of the method is as follows: the determination of the characteristic absorption of peptide bonds under far-UV light is independent of the chromophore content, with few interfering substances and simple operation, which is suitable for detecting human collagen and analogues thereof which are not colored by Coomassie Brilliant Blue (the reference document is Walker JM. The Protein Protocols Handbook, second edition. Humana Press. 43-45). After the detection of the protein concentration, the concentration of all the proteins to be detected was adjusted to 0.5 mg/ml with PBS.
2) 100 µL of various protein solutions and blank PBS control solution were added to a 96-well plate, which was placed at room temperature for 60 minutes.
3) 10⁵ 3T3 cells (from Teacher Pei TONG, Tsinghua University) were added to each well, and incubated at 37°C for 60 minutes.
4) Each well was washed with PBS 4 times.
5) The absorbance of OD₄₉₂ₙₘ was detected by LDH detection kit (Roche, 04744926001). According to the value of the blank control, the cell adhesion rate could be calculated. The calculation formula is as follows: cell adhesion rate = (test cell-blank well) × 100%/(positive well-blank well). The adhesion rate of cells can reflect the activity of collagen. The higher the activity of the protein, the better the external environment can be provided for the cells in a short time, which helps the cells to adhere to the wall.

The results are shown in FIG. 2. In view of the comparison, as compared with the commercialized human collagen, the recombinant type-III collagen C1S4T and the recombinant type-III collagen C1S5T, the recombinant type-I humanized collagen TC1R4 provided by the present disclosure, has superior bio-adhesive activity.

## Claims

1. A recombinant type-I humanized collagen polypeptide,
wherein the recombinant type-I humanized collagen polypeptide comprises n repeats of a sequence set forth in SEQ ID No: 1, n being an integer greater than or equal to 1, wherein the repetitive sequences are directly linked to each other when n is an integer greater than or equal to 2; optionally, the N-terminus of the recombinant type-I humanized collagen polypeptide comprises an amino acid sequence capable of being cleaved off by TEV protease.

2. The recombinant type-I humanized collagen polypeptide according to item 1, wherein the amino acid sequence capable of being cleaved off by TEV protease comprises a sequence set forth in SEQ ID No:4; preferably, the sequence set forth in SEQ ID No:4 is directly linked to the sequence set forth in SEQ ID No: 1.

3. The recombinant type-I humanized collagen polypeptide according to item 1 or 2, wherein the recombinant type-I humanized collagen polypeptide comprises:
a) an amino acid sequence set forth in SEQ ID No:3;
b) an amino acid sequence having 90%, 92%, 95%, 96%, 97%, 98% or 99% identity with the amino acid sequence set forth in SEQ ID No:3, which retains the cell adhesion effect of the amino acid sequence set forth in SEQ ID No:3;
c) an amino acid sequence obtained by adding, substituting, deleting or inserting one or more amino acid residues into the amino acid sequence set forth in SEQ ID No:3, which retains the cell adhesion effect of the amino acid sequence set forth in SEQ ID No:3; or
d) an amino acid sequence encoded by a nucleotide sequence which hybridizes with a polynucleotide sequence encoding the amino acid sequence set forth in SEQ ID No:3 under a stringency condition, and the amino acid sequence retains the cell adhesion effect of the amino acid sequence set forth in SEQ ID No:3, the stringency condition is a medium stringency condition, medium-high stringency condition, high stringency condition or very high stringency condition.

4. A polynucleotide encoding the recombinant type-I humanized collagen polypeptide according to any one of items 1 to 3.

5. An expression vector comprising the polynucleotide according to item 4.

6. A host cell comprising the expression vector according to item 5.

7. A method for producing the recombinant type-I humanized collagen polypeptide according to any one of items 1 to 3, which includes steps of:
1) culturing the host cell according to item 6 in a culture medium and producing a polypeptide;
2) harvesting and purifying the polypeptide, preferably purifying the polypeptide by Ni column and/or anion exchange chromatography; and
3) optionally enzymatically cleaving the polypeptide, preferably enzymatically cleaving the polypeptide with TEV protease.

8. Use of the recombinant type-I humanized collagen polypeptide according to any one of items 1 to 3 in the preparation of a product, wherein the product preferably is a tissue engineering product, a cosmetic, a health product or a medicine.

9. A product comprising the recombinant type-I humanized collagen polypeptide according to any one of items 1 to 3, wherein the product preferably is a tissue engineering product, a cosmetic, a health product or a medicine.

10. Use of the recombinant type-I humanized collagen polypeptide according to any one of items 1 to 3 in the preparation of a product having a function of promoting cell adhesion.
